(19) Europäisches Patentamt
European Patent Office
Office européen des brevets

(11) Veröffentlichungsnummer: **0 179 020**
**A2**

(12) **EUROPÄISCHE PATENTANMELDUNG**

(21) Anmeldenummer: 85810466.4

(22) Anmeldetag: 14.10.85

(51) Int. Cl.[4]: C 07 D 409/04
A 01 N 43/40

(30) Priorität: 19.10.84 CH 5010/84
19.10.84 CH 5011/84
05.09.85 CH 3830/85

(43) Veröffentlichungstag der Anmeldung:
23.04.86 Patentblatt 86/17

(84) Benannte Vertragsstaaten:
BE CH DE FR GB IT LI NL

(71) Anmelder: CIBA-GEIGY AG
Klybeckstrasse 141
CH-4002 Basel(CH)

(72) Erfinder: Traber, Walter, Dr.
Neueneichweg 12
CH-4153 Reinach(CH)

(72) Erfinder: Fischer, Hanspeter, Dr.
Burggartenstrasse 14
CH-4103 Bottmingen(CH)

(54) **Thioxanthene in der Schädlingsbekämpfung.**

(57) Die Verwendung in der Schädlingsbekämpfung von Thioxanthen-9-yliden-piperidinen der Formel I

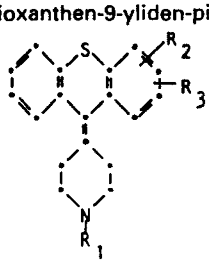

(I)

und deren Salze,

worin

$R_1$ Wasserstoff, $C_1$–$C_4$-Alkyl, $C_3$–$C_6$-Alkenyl, $C_3$–$C_5$-Alkinyl Cyano oder $-COR_4$,

$R_2$ und $R_3$ unabhängig voneinander Wasserstoff, Halogen, $C_1$–$C_4$-Alkyl, $C_1$–$C_4$-Alkoxy, $C_1$–$C_4$-Halogenalkyl oder Nitro,

$R_4$ 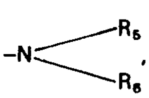, $C_1$–$C_4$-Alkyl, $C_1$–$C_4$-Alkoxy-$C_1$–$C_4$-alkyl,

$C_1$ –$C_4$-Halogenalkyl, $C_3$–$C_6$-Cycloalkyl,

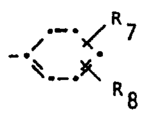 , 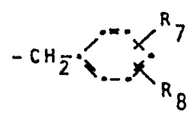 oder $C_1$-$C_4$-Alkoxycarbonyl,

$R_5$ und $R_6$ unabhängig voneinander Wasserstoff, $C_1$–$C_4$-Alkyl oder $C_1$–$C_4$-Alkoxy und

$R_7$ und $R_8$ unabhängig voneinander Wasserstoff, Halogen, $C_1$–$C_4$-Alkyl, $C_1$–$C_4$-Alkoxy oder Nitro bedeuten.

Ebenso werden die neuen Verbindungen und deren Salze der Formel Ia

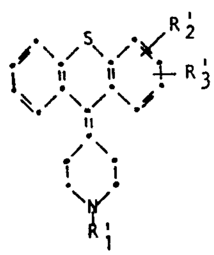

(Ia)

./...

EP 0 179 020 A2

worin
$R_1$ Wasserstoff,
$R_2'$ Brom, $C_2$–$C_4$-Alkyl, $C_2$–$C_4$-Alkoxy, $-CHF_2$ oder Nitro und
$R_3'$ Wasserstoff, Halogen, $C_1$–$C_4$-Alkyl, $C_1$–$C_4$-Alkoxy, $C_1$–$C_4$-Halogenalkyl order Nitro oder
$R_1'$ Aethyl, Propyl, Isopropyl, n–, i–, sek.– oder tert. Butyl und
$R_2'$ und $R_3'$ unabhängig voneinander Wasserstoff, Fluor, Brom, $C_1$–$C_4$-Alkyl, $C_1$–$C_4$-Alkoxy, $-CHF_2$ oder Nitro oder
$R_1'$ Propyl order Isopropyl,
$R_2'$ Chlor und
$R_3'$ Wasserstoff, Halogen, $C_1$–$C_4$-Alkyl, $C_1$–$C_4$-Alkoxy, $C_1$–$C_4$-Halogenalkyl oder Nitro order
$R_1'$ Cyano und
$R_2'$ und $R_3'$ Wasserstoff oder
$R_1'$ $-COR_4'$ oder $-CH_2-C{\equiv}CH$,
$R_2'$ und $R_3'$ unabhängig voneinander Wasserstoff, Halogen, $C_1$–$C_4$-Alkyl, $C_1$–$C_4$-Alkoxy, $C_1$–$C_4$-Halogenalkyl oder Nitro,

$R_4'$ 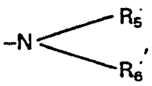 , $C_1$–$C_4$-Alkyl, $C_1$–$C_4$-Halogenalkyl, $C_3$–$C_8$-Cycloalkyl,

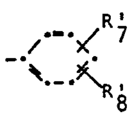 , 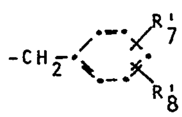 oder Carboxyloxy-$C_1$-$C_4$-alkyl,

$R_5'$ und $R_6'$ unabhängig voneinander Wasserstoff, $C_1$–$C_4$-Alkyl oder $C_1$–$C_4$-Alkoxy und
$R_8'$ und $R_8'$ unabhängig voneinander Wasserstoff, Halogen, $C_1$–$C_4$-Alkyl, $C_1$–$C_4$-Alkoxy oder Nitro oder
$R_1'$ $-CH_2-CH=CH_2$ und
$R_2$ $R_3'$ unabhängig voneinander Wasserstoff, Halogen, $C_1$–$C_4$-Alkyl, $C_1$–$C_4$-Alkoxy oder Nitro bedeuten und

Schädlingsbekämpfungsmittel, welche als aktive Komponente Verbindungen der Formeln I und Ia enthalten sowie ein Verfahren zur Herstellung dieser Thioxanthen–9-ylidenpiperidinen beschrieben.

CIBA-GEIGY AG
Basel (Schweiz)

5-15118/119/1+2

## Thioxanthene in der Schädlingsbekämpfung

Die vorliegende Erfindung betrifft die Verwendung von Thioxanthen-9-yliden-piperidinen und deren Salze in der Schädlingsbekämpfung, sowie Mittel, welche als aktive Komponente solche Thioxanthen-9-yliden-piperidine enthalten und Verfahren zu ihrer Herstellung. Die erfindungsgemäss zu verwendenden Thioxanthen-9-yliden-piperidine haben die Formel

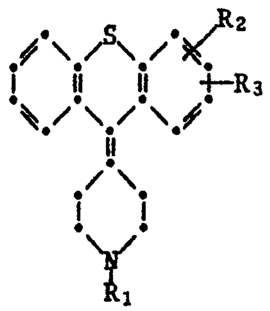

(I),

worin

$R_1$ Wasserstoff, $C_1$-$C_4$-Alkyl, $C_3$-$C_5$-Alkenyl, $C_3$-$C_5$-Alkinyl, Cyano oder $-COR_4$,

$R_2$ und $R_3$ unabhängig voneinander Wasserstoff, Halogen, $C_1$-$C_4$-Alkyl, $C_1$-$C_4$-Alkoxy, $C_1$-$C_4$-Halogenalkyl oder Nitro,

$R_4$ 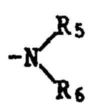 , $C_1$-$C_4$-Alkyl, $C_1$-$C_4$-Alkoxy-$C_1$-$C_4$-alkyl,

$C_1$-$C_4$-Halogenalkyl, $C_3$-$C_6$-Cycloalkyl,

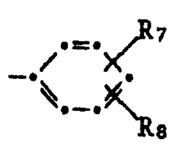 , $-CH_2-$ 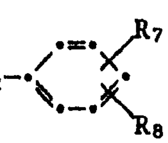 oder $C_1$-$C_4$-Alkoxycarbonyl,

$R_5$ und $R_6$ unabhängig voneinander Wasserstoff, $C_1$-$C_4$-Alkyl oder $C_1$-$C_4$-Alkoxy und

$R_7$ und $R_8$ unabhängig voneinander Wasserstoff, Halogen, $C_1$-$C_4$-Alkyl, $C_1$-$C_4$-Alkoxy oder Nitro bedeuten.

Für die Salzbildung bei den Verbindungen der Formel I kommen anorganische Säuren wie beispielsweise HCl, $H_2SO_4$, HBr und $H_3PO_4$ und organische Säuren beispielsweise gesättigte und ungesättigte Mono-, Di- und Tricarbonsäuren wie z.B. Ameisensäure, Essigsäure, Oxalsäure, Phthalsäure, Bernsteinsäure und Zitronensäure in Betracht.

Unter Halogen ist dabei Fluor, Chlor, Brom oder Jod, insbesondere aber Chlor oder Fluor, zu verstehen.

Die bei $R_1$, $R_2$ und $R_3$ in Frage kommenden Alkyl-, Alkoxy-, Alkenyl-, Alkinyl- und Halogenalkylgruppen können geradkettig oder verzweigt sein. Beispiele von solchen Gruppen sind u.a.: Methyl, Methoxy, Trifluormethyl, Aethyl, Aethoxy, Propyl, Isopropyl, n-, i-, sek.-und tert. Butyl, $-CH_2-CH=CH_2$, $-CH_2-C\equiv CH$.

Beispiele von Cycloalkylgruppen bei $R_4$ sind Cyclopropyl und Cyclohexyl.

Gegenstand der Erfindung sind auch die neuen Verbindungen und deren Salze der Formel

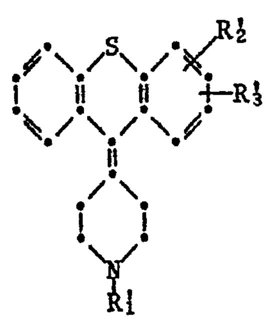

(Ia),

worin

$R_1'$ Wasserstoff,

$R_2'$ Brom, $C_2$-$C_4$-Alkyl, $C_2$-$C_4$-Alkoxy, $-CHF_2$ oder Nitro und

$R_3'$ Wasserstoff, Halogen, $C_1$-$C_4$-Alkyl, $C_1$-$C_4$-Alkoxy, $C_1$-$C_4$-Halogenalkyl oder Nitro oder

$R_1'$ Aethyl, Propyl, Isopropyl, n-, i-, sek.- oder tert.Butyl und

$R_2'$ und $R_3'$ unabhängig voneinander Wasserstoff, Fluor, Brom, $C_1$-$C_4$-Alkyl, $C_1$-$C_4$-Alkoxy, $-CHF_2$ oder Nitro oder

$R_1'$ Propyl oder Isopropyl,

$R_2'$ Chlor und

$R_3'$ Wasserstoff, Halogen, $C_1$-$C_4$-Alkyl, $C_1$-$C_4$-Alkoxy, $C_1$-$C_4$-Halogenalkyl oder Nitro oder

$R_1'$ Cyano und

$R_2'$ und $R_3'$ Wasserstoff oder

$R_1'$ - $COR_4'$ oder $-CH_2-C{\equiv}CH$,

$R_2'$ und $R_3'$ unabhängig voneinander Wasserstoff, Halogen, $C_1$-$C_4$-Alkyl, $C_1$-$C_4$-Alkoxy, $C_1$-$C_4$-Halogenalkyl oder Nitro,

$R_4'$ 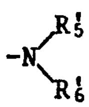 , $C_1$-$C_4$-Alkyl, $C_1$-$C_4$-Halogenalkyl, $C_3$-$C_6$-Cycloalkyl,

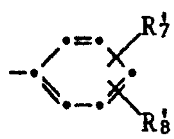 , $-CH_2-$ 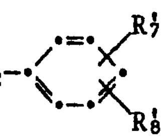 oder Carboxyloxy-$C_1$-$C_4$-alkyl,

$R_5'$ und $R_6'$ unabhängig voneinander Wasserstoff, $C_1$-$C_4$-Alkyl oder $C_1$-$C_4$-Alkoxy und

$R_7'$ und $R_8'$ unabhängig voneinander Wasserstoff, Halogen, $C_1$-$C_4$-Alkyl, $C_1$-$C_4$-Alkoxy oder Nitro oder

$R_1'$ $-CH_2-CH{=}CH_2$ und

$R_2'$ und $R_3'$ unabhängig voneinander Wasserstoff, Halogen, $C_1$-$C_4$-Alkyl, $C_1$-$C_4$-Alkoxy oder Nitro bedeuten.

Die Verbindungen der Formeln I und Ia werden nach an sich bekannten Methoden z.B. wie folgt hergestellt.

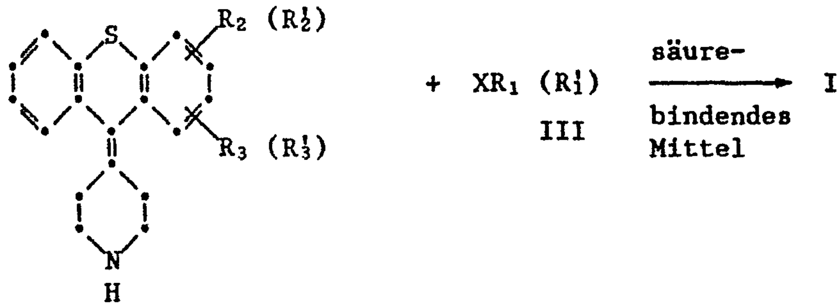


II

+ $XR_1$ ($R_1^1$) III → I

In den Formeln II und III haben $R_1$, $R_2$ und $R_3$ die für die Formel I und $R_1^1$, $R_2^1$ und $R_3^1$ die für die Formal Ia angegebene Bedeutung.

In der Formel III steht X für ein Halogenatom, insbesondere für Chlor. Als säurebindende Mittel kommen z.B. tertiäre Amine, wie Trialkylamine und Pyridin, ferner Hydride, Hydroxide, Oxide, Carbonate und Bicarbonate von Alkali- und Erdalkalimetallen sowie Alkalkimetallalkoholate, wie z.B. Kalium-t.-butylat und Natriummethylat in Betracht.

Das Verfahren wird bei einer Reaktionstemperatur zwischen -10° bis 150°C, meist zwischen 20° und 80°C, bei normalem Druck und vorzugsweise in einem inerten Lösungs- oder Verdünnungsmittel durchgeführt. Als Lösungs- oder Verdünnungsmittel eignen sich z.B. Aether und ätherartige Verbindungen, wie Diäthyläther, Dipropyläther, Dioxan, Dimethoxyäthan und Tetrahydrofuran; Amide, wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton und Methyläthylketon.

Die Verbindungen der Formeln II und III sind bekannt und können analog bekannten Methoden hergestellt werden.

Aus der französischen Patentschrift Nr. 2 290 202 ist es bekannt, dass Verbindungen der Formel I pharmazeutische Wirkungen haben.

Ueberraschenderweise wurde nun gefunden, dass sich Verbindungen der Formeln I und Ia auch zur Bekämpfung von Schädlingen an Tieren und Pflanzen sowie von phythopathogenen Pilzen eignen.

So wirken Verbindungen der Formel I bzw. Ia gegen die in den folgenden Klassen angehörenden phytopathogenen Pilze: Ascomycetes (z.B. Erysiphaceae, Fusarium, Helminthosporium); Basidiomycetes wie Puccinia, Rhizoctonia, Tilletia, Hemileia; Fungi imperfecti (z.B. Cercospora, Botrytis, Septoria); Phycomycetes wie Phytophthora. Ueberdies wirken die Verbindungen der Formeln I bzw. Ia systemisch. Die Verbindungen der Formeln I bzw. Ia werden vorteilhaft als Beizmittel zur Behandlung von Saatgut und Vorrat (Früchte, Knollen, Körner) und Pflanzenstecklingen zum Schutz von Pilzinfektionen sowie gegen im Erdboden auftretende phytopathogene Pilze eingesetzt.

Insbesondere eigenen sich die Verbindungen der Formel I bzw. Ia zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und die Milben und Zecken der Ordnung Akarina.

Vor allem eignen sich Verbindungen der Formel I bzw. Ia zur Bekämpfung von pflanzenschädigenden Insekten, insbesondere pflanzenschädigenden Frassinsekten, in Zier- und Nutzpflanzen, insbesondere in Baumwoll- und Reiskulturen (z.B. Spodoptera littoralis, Heliothis virescens, Chilo suppressalis und Laodelphax) sowie Gemüse- und Obstkulturen (z.B. Leptinotarsa decemlineata, Myzus persicae, Laspeyresia pomonella und Adoxophyes reticulana) und von Bodeninsekten (z.B. Aulacophora femoralis, Chortophila brassicae, Diabrotica balteata, Pachnoda savignyi und Scotia ypsilon).

Wirkstoffe der Formel I bzw. Ia zeigen auch eine sehr günstige Wirkung gegen Fliegen, wie z.B. Musca domestica und Mückenlarven. Sie beeinflussen das Verhalten der Insekten, insbesondere das Frassverhalten und die Flugorientierung.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; pyrethrinartige Verbindungen sowie Karbamate und chlorierte Kohlenwasserstoffe.

Mit besonderem Vorteil werden Verbindungen der Formel I bzw. Ia auch mit Substanzen kombiniert, welche einen synergistischen oder verstärkenden Effekt z.B. auf die Pheromonperzeption ausüben. Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-(octylsulfinyl)-propyl)-benzol.

Die Verbindungen der Formel I bzw. Ia werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältissen entsprechend gewählt.

Die Formulierungen, d.h. die den Wirkstoff der Formel I bzw. Ia und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder äthyläther, Essigester, Propylmyristat oder Propylpalmitat, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; Silikonöle oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I bzw. Ia nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eigenen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talöl gewonnen werden können. Ferner sind auch die Fettsäuremethyltaurin-salze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca oder Triäthanolaminsalze der Dodecylbenzolsulfonsäure, der Dibutylnaphthalinsulfonsäure, oder eines Nahphtalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nichtionische Tenside kommen in erster Linie Polyglykolätherderivate von aliphatischen oder cycloaliphatischen Alkohlen, gesättigten oder ungesättigten Fettsäuren und Alkylphenolen in Frage, die 3 bis 30 Glykolätherrgruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltend Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5 Aethylenglykoleinheiten.

Als Beispiele nichtionischer Tenside seien Nonylphenolpolyäthoxyäthanole, Ricinusölpolyglykoläther, Ricinusölthioxilat, Polypropylenpolyäthylenoxid-Addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi-(2-chloräthyl)-äthylammoniumbromid.

Die in der Formulierungstechnik gebräuchlichen Tenside sind u.a. in folgenden Publlikationen beschrieben:

"Mc Cutcheon's Detergents and Emulsifiers Annual" MC Publishing Corp., Ridgewood, New Jersey, 1982; Dr. Helmut Stache: "Tensid Taschenbuch" Carl Hanser Verlag München/Wien 1981.

Die pestiziden Zubereitungen enthalten in der Regel 0,1 bis 99 %, insbesondere 0,1 bis 95 %, Wirkstoff der Formel I bzw. Ia, 1 bis 99,9 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung der Verbindung Nr. 1 der Formel

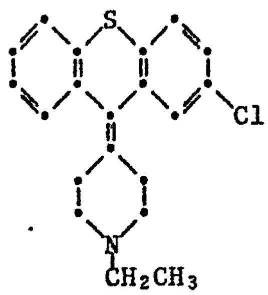

Zu einer Lösung von 6,3 g 4-(2-Chlor-thioxanthen-9-yliden)-piperidin in 70 ml Tetrahydrofuran tropft man bei einer Temperatur von 21°C während 20 Minuten eine Lösung von 0,66 g Natriumhydrid in 30 ml Tetrahydrofuran. Nach 22-stündigem Kochen am Rückfluss und nach 48-stündigem Stehenlassen bei Raumtemperatur gibt man auf einmal 3,12 g Aethyljodid zu. Nach nochmaligem 24-stündigem Kochen am Rückfluss giesst man die Reaktionslösung auf 400 g Eiswasser und zieht das Produkt mit Methylenchlorid aus. Die organische Phase wird abgetrennt, über Magnesiumsulfat getrocknet und eingeengt.

Nach Chromatographieren des Rohproduktes über Kieselgel mit Toluol/Essigester (95:5) als Eluiermittel erhält man die Titelverbindung mit einem Schmelzpunkt von 55-58°C.

Auf analoge Weise werden auch folgende Verbindungen hergestellt:

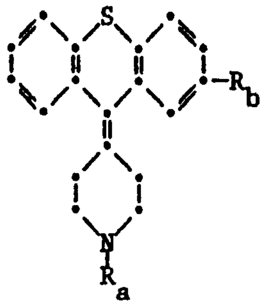

| Nr. | $R_a$ | $R_b$ | Schmelzpunkt |
|---|---|---|---|
| 2 | $CH_3$ | H | 120-121°C |
| 3 | $CH_3$ | Cl | 107-109°C |
| 4 | CN | H | 194-196°C |
| 5 | H | H | 143-145°C |
| 6 | CN | Cl | 155-157°C |
| 7 | H | Cl | 142-143°C |
| 8 | $C_3H_7(n)$ | H | 156-157°C |
| 9 | $C_2H_5$ | H | 105-107°C |
| 10 | $C_3H_7(i)$ | Cl | 60°C |
| 11 | $C_3H_7(n)$ | Cl | 68- 72°C |
| 12 | 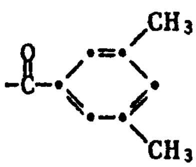 | H | 94- 97°C |
| 13 | $-CH_2-CH=CH_2$ | H | 131-133°C |
| 14 | $-CH_2-CH=CH_2$ | Cl | 120-122°C |
| 15 | $-CH_2-C\equiv CH$ | H | 53- 56°C |
| 16 | $-CH_2-C\equiv CH$ | Cl | 57- 59°C |
| 17 | 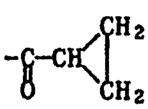 | H | 190-192°C |
| 18 | 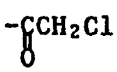 | H | 126-128°C |
| 19 | 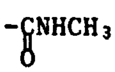 | H | 229-231°C |
| 20 | 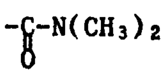 | H | 127-129°C |

| Nr. | $R_a$ | $R_b$ | Schmelzpunkt |
|---|---|---|---|
| 21 | 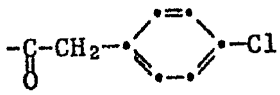 | Cl | 119-121°C |
| 22 | 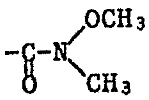 | Cl | 152-155°C |
| 23 | $-\underset{O}{\underset{\|}{C}}-CH_2OCH_3$ | Cl | 76- 79°C |
| 24 | $-\underset{O}{\underset{\|}{C}}-\underset{O}{\underset{\|}{C}}-OC_2H_5$ | H | 118-120°C |
| 25 | $-\underset{O}{\underset{\|}{C}}-\underset{CH_3}{\underset{\|}{C}}H-C_2H_5$ | Cl | 86- 88°C |
| 26 | $-\underset{O}{\underset{\|}{C}}NH_2$ | H | 241-243°C |
| 27 | $CH_3$ | $CF_3$ | 100-101°C |

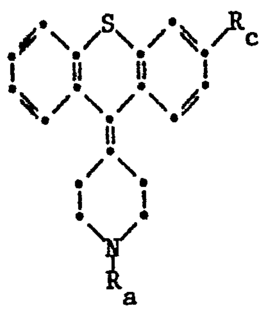

| Nr. | $R_a$ | $R_c$ | Schmelzpunkt |
|---|---|---|---|
| 28 | $CH_3$ | Cl | 134-145°C |
| 29 | $CH_3$ | $CH_3$ | 60°C |

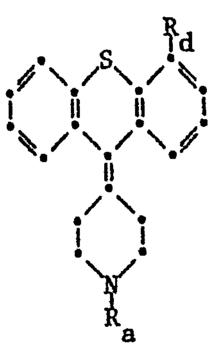

| Nr. | $R_a$ | $R_d$ | Schmelzpunkt |
|---|---|---|---|
| 30 | $CH_3$ | Cl | 102-104°C |

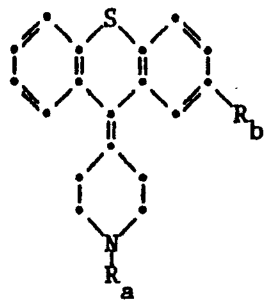

Salze

| Nr. | $R_a$ | $R_b$ | | Schmelzpunkt |
|---|---|---|---|---|
| 31 | $CH_3$ | H | $\cdot HCl$ | 153-155°C |
| 32 | $CH_3$ | $CH_3$ | $\cdot HBr$ | 189-181°C |
| 33 | $CH_3$ | H | $\cdot CF_3COOH$ | 205°C |

<u>Beispiel 2</u>: <u>Formulierungsbeispiele für Wirkstoffe der Formeln I und Ia gemäss dem Herstellungsbeispiel 1</u>

(% = Gewichtsprozent)

| 2.1 Emulsions-Konzentrate | a) | b) | c) | d) |
|---|---|---|---|---|
| Wirkstoff gemäss dem Herstellungsbeispiel 1 | 10 % | 25 % | 40 % | 50 % |
| Ca-Dodecylbenzolsulfonat | - | 5 % | 8 % | 6 % |
| Ricinusöl-polyäthylenglykoläther (36 Mol AeO) | - | 5 % | - | - |
| Tributylphenol-polyäthylenglykoläther (30 Mol AeO) | - | - | 12 % | 4 % |
| Ricinusölthioxilat | 25 % | - | - | - |
| Cyclohexanon | - | - | 15 % | 20 % |
| Butanol | 15 % | - | - | - |
| Xylolgemisch | - | 65 % | 25 % | 20 % |
| Essigester | 50 % | - | - | - |

Aus solchen Konzentraten können durch Verdünnen mit Wasser Emulsionen jeder gewünschten Konzentration hergestellt werden.

| 2.2 Lösungen | a) | b) |
|---|---|---|
| Wirkstoff gemäss dem Herstellungsbeispiel 1 | 10 % | 5 % |
| Aethylenglykol-monomethyläther | - | - |
| Polyäthylenglykol (MG 400) | 70 % | - |
| N-Methyl-2-pyrrolidon | 20 % | - |
| Epoxidiertes Kokosnussöl | - | 1 % |
| Benzin (Siedegrenzen 160-190°C) | - | 94 % |

Die Lösungen sind zur Anwendung in Form kleinster Tropfen geeignet.

| 2.3 Granulate | a) | b) |
|---|---|---|
| Wirkstoff gemäss dem Herstellungsbeispiel 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) | c) | d) |
|---|---|---|---|---|
| Wirkstoff gemäss dem Herstellungsbeispiel 1 | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff erhält man gebrauchsfertige Stäubemittel.

**2.5 Spritzpulver**

| | a) | b) | c) |
|---|---|---|---|
| Wirkstoff gemäss dem Herstellungsbeispiel 1 | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

**2.6 Extruder Granulat**

| | |
|---|---|
| Wirkstoff gemäss dem Herstellungsbeispiel 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

**2.7 Umhüllungs-Granulat**

| | |
|---|---|
| Wirkstoff gemäss dem Herstellungsbeispiel 1 | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

2.8 Suspensions-Konzentrat

| | |
|---|---|
| Wirkstoff gemäss dem | |
| Herstellungsbeispiel 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen | |
| wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Biologiebeispiele für Wirkstoffe gemäss dem Herstellungsbeispiel 1

3.1 Insektizide Frassgift-Wirkung: Spodoptera littoralis

Baumwollpflanzen werden mit einer Versuchslösung, enthaltend 400 ppm der zu prüfenden Verbindung, besprüht.

Nach dem Antrocknen des Belages werden die Pflanzen mit Larven von Spodoptera littoralis ($L_1$-Stadium) besetzt. Man verwendet pro Versuchsverbindung zwei Pflanzen. Die Auswertung der erzielten Abtötungsrate erfolgt nach 2, 4, 24, 48 und 72 Stunden. Der Versuch wird bei 28°C und 60 % relativer Luftfeuchtigkeit durchgeführt. Verbindungen gemäss Beispiel 1 zeigen im obigen Test 80-100%ige Wirkung gegenüber Spodoptera littoralis-Larven.

3.2 Wirkung gegen Lucilia sericata

Zu 9 ml eines Zuchtmediums wird bei 50°C ein ml einer 0,1 % Aktivsubstanz enthaltenden wässrigen Zubereitung hinzugefügt. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt. Verbindungen gemäss Beispiel 1 zeigen in diesem Test 80-100%ige Wirkung gegen Lucilia sericata.

3.3 Wirkung gegen Aëdes aegypti

Auf die Oberfläche von 150 ml Wasser, das sich in einem Behälter befindet, wird so viel einer 0,1%igen acetonischen Lösung des Wirkstoffs pipettiert, dass eine Konzentration von 12,5 ppm erhalten wird. Nach Verdunsten des Acetons wird der Behälter mit 30 bis 40 2tägigen Aëdes-Larven beschickt. Nach 2 und 7 Tagen wird die % Mortalität (Anzahl der schwimmunfähigen Larven) geprüft.

Verbindungen gemäss Beispiel 1 zeigen 100%ige Wirkung (Mortalität) im obigen Test.

3.4 Ovizide Wirkung auf Heliothis virescens

Entsprechende Mengenanteile einer benetzbaren pulverförmigen Formulierung, enthaltend 25 Gew.% des zu prüfenden Wirkstoffes, werden mit jeweils so viel Wasser vermischt, dass sich wässrige Emulsionen mit Wirkstoffkonzentrationen von 400 bis 12,5 ppm ergeben.

In diese wirkstoffhaltigen Emulsionen werden eintägige Eigelege von Heliothis auf Cellophan® während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgelegt. Zur Auswertung wird die zur 100%igen Abtötung der Eier erforderliche Wirkstoffkonzentration bestimmt.

Verbindungen gemäss Beipiel 1 zeigen bei 12,5 ppm 100%ige Wirkung (Mortalität) in obigem Test.

3.5 Insektizide Kontaktwirkung: Myzus persicae

Etwa 4 cm hohe, in Wasser angezogene Erbsenkeimlinge werden vor Versuchsbeginn je mit ca. 200 Individuen der Spezies Myzus persicae besiedelt. Die so behandelten Pflanzen werden 24 Stunden später mit einer wässrigen Suspension enthaltend 400 ppm der zu prüfenden Verbindung bis zur Tropfnässe besprüht. Man verwendet pro Konzentration zwei Pflanzen. Eine Auswertung der erzielten Abtötungsrate erfolgt 48 Stunden nach Applikation. Der Versuch wird bei 20-22°C und 60 % relativer Luftfeuchtigkeit durchgeführt.

Verbindungen gemäss Beispiel 1 zeigen in diesem Test eine 80-100%ige Wirkung.

3.6 Wirkung gegen Bodeninsekten (Diabrotica balteata)

Es werden 5 etwa einen bis drei cm lange Maiskeimlinge sowie eine Rondelle aus Filterpapier in eine 0,2 bis 12,5 ppm des zu prüfenden Wirkstoffes enthaltende wässrige Zubereitung getaucht. Die feuchte Filterpapierrondelle wird auf dem Boden eines 200 ml Plastikbechers ausgelegt, und dann werden die 5 behandelten Maiskeimlinge zusammen mit 10 Larven von Diabrotica balteata des zweiten bis dritten Larvalstadiums in den Becher gegeben. Pro Wirkstoffkonzentration werden 2 Ansätze durchgeführt. Die mit den Larven besetzten Becher werden während 6 Tagen bei Tageslicht, einer relativen Luftfeuchtigkeit von 40 bis 60 % und einer Temperatur von 22 bis 24°C gehalten. Danach wird die prozentuale Abtötung der Testtiere bestimmt.

Verbindungen gemäss Beispiel 1 zeigen bei einer Wirkstoffkonzentration von 12,5 ppm in diesem Test 100%ige Wirkung.

3.7 Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung enthaltend 400 ppm des zu prüfenden Wirkstoffes eingetaucht. Nach dem Antrocknen der Lösung werden die Eier in Petrischalen ausgelegt und bei einer Temperatur von 28°C belassen. Nach 6 Tagen wird der prozentuale Schlupf aus den behandelten Eiern bewertet.

Verbindungen gemäss Beispiel 1 zeigen 100%ige Wirkung in obigem Test.

Patentansprüche

1. Ein Schädlingsbekämpfungsmittel, welches als aktive Komponente eine Verbindung der Formel I

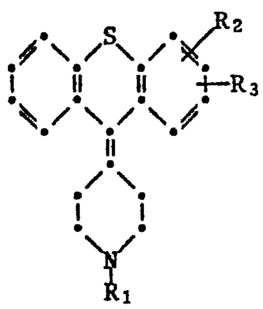

(I)

und deren Salze enthält, worin

$R_1$ Wasserstoff, $C_1$-$C_4$-Alkyl, $C_3$-$C_5$-Alkenyl, $C_3$-$C_5$-Alkinyl, Cyano oder $-COR_4$,

$R_2$ und $R_3$ unabhängig voneinander Wasserstoff, Halogen, $C_1$-$C_4$-Alkyl, $C_1$-$C_4$-Alkoxy, $C_1$-$C_4$-Halogenalkyl oder Nitro,

$R_4$ 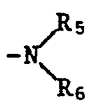 , $C_1$-$C_4$-Alkyl, $C_1$-$C_4$-Alkoxy-$C_1$-$C_4$-alkyl,

$C_1$-$C_4$-Halogenalkyl, $C_3$-$C_6$-Cycloalkyl,

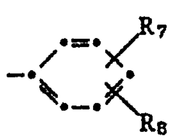 , 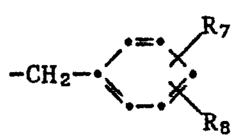 oder $C_1$-$C_4$-Alkoxycarbonyl,

$R_5$ und $R_6$ unabhängig voneinander Wasserstoff, $C_1$-$C_4$-Alkyl oder $C_1$-$C_4$-Alkoxy und

$R_7$ und $R_8$ unabhängig voneinander Wasserstoff, Halogen, $C_1$-$C_4$-Alkyl, $C_1$-$C_4$-Alkoxy oder Nitro bedeuten.

2. Ein Schädlingsbekämpfungsmittel gemäss Anspruch 1, welches als aktive Komponente eine Verbindung der Formel Ia

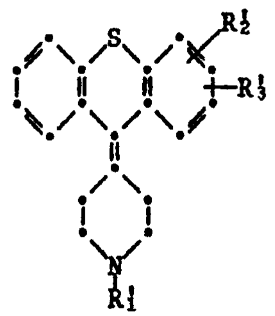

(Ia)

enthält, worin

$R_1'$ Wasserstoff,

$R_2'$ Brom, $C_2$-$C_4$-Alkyl, $C_2$-$C_4$-Alkoxy, $-CHF_2$ oder Nitro und

$R_3'$ Wasserstoff, Halogen, $C_1$-$C_4$-Alkyl, $C_1C_4$-Alkoxy, $C_1$-$C_4$-Halogenalkyl oder Nitro oder

$R_1'$ Aethyl, Propyl, Isopropyl, n-, i-, sek.- oder tert.Butyl und

$R_2'$ und $R_3'$ unabhängig voneinander Wasserstoff, Fluor, Brom, $C_1$-$C_4$-Alkyl, $C_1$-$C_4$-Alkoxy, $-CHF_2$ oder Nitro oder

$R_1'$ Propyl oder Isopropyl,

$R_2'$ Chlor und

$R_3'$ Wasserstoff, Halogen, $C_1$-$C_4$-Alkyl, $C_1$-$C_4$-Alkoxy, $C_1$-$C_4$-Halogenalkyl oder Nitro oder

$R_1'$ Cyano und

$R_2'$ und $R_3'$ Wasserstoff oder

$R_1'$ - $COR_4'$ oder $-CH_2-C{\equiv}CH$,

$R_2'$ und $R_3'$ unabhängig voneinander Wasserstoff, Halogen, $C_1$-$C_4$-Alkyl, $C_1$-$C_4$-Alkoxy, $C_1$-$C_4$-Halogenalkyl oder Nitro,

$R_4'$ 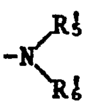 , $C_1$-$C_4$-Alkyl, $C_1$-$C_4$-Halogenalkyl, $C_3$-$C_6$-Cycloalkyl,

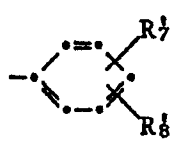 , $-CH_2-$ 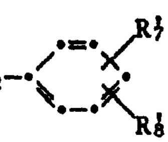 oder Carboxyloxy-$C_1$-$C_4$-alkyl,

$R_5'$ und $R_6'$ unabhängig voneinander Wasserstoff, $C_1$-$C_4$-Alkyl oder $C_1$-$C_4$-Alkoxy und

$R_7'$ und $R_8'$ unabhängig voneinander Wasserstoff, Halogen, $C_1$-$C_4$-Alkyl, $C_1$-$C_4$-Alkoxy oder Nitro oder

$R_1'$ $-CH_2-CH{=}CH_2$ und

$R_2^1$ und $R_3^1$ unabhängig voneinander Wasserstoff, Halogen, $C_1$-$C_4$-Alkyl, $C_1$-$C_4$-Alkoxy oder Nitro bedeuten.

3. Ein Schädlingsbekämpfungsmittel gemäss Anspruch 1, welches als aktive Komponente die Verbindung der Formel

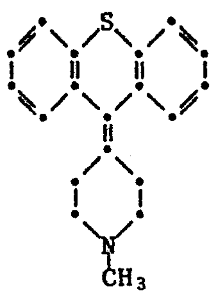

enthält.

4. Verwendung von Schädlingsbekämpfungsmitteln gemäss Anspruch 1 zur Bekämpfung von Schädlingen an Tieren und Pflanzen sowie im Boden.

5. Die Verwendung gemäss Anspruch 4 zur Bekämpfung von Insekten und Vertretern der Ordnung Akarina.

6. Die Verwendung gemäss Anspruch 4 zur Bekämpfung von phytopathogenen Pilzen.

7. Ein Thioxanthen-9-yliden-piperidin und dessen Salze der Formel Ia

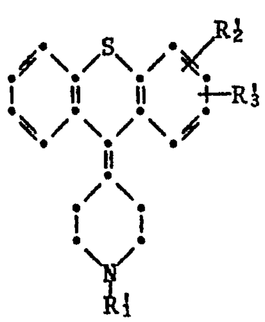

(Ia),

 

worin

$R_1'$ Wasserstoff,

$R_2'$ Brom, $C_2$-$C_4$-Alkyl, $C_2$-$C_4$-Alkoxy, $-CHF_2$ oder Nitro und

$R_3'$ Wasserstoff, Halogen, $C_1$-$C_4$-Alkyl, $C_1C_4$-Alkoxy, $C_1$-$C_4$-Halogenalkyl oder Nitro oder

$R_1'$ Aethyl, Propyl, Isopropyl, n-, i-, sek.- oder tert.Butyl und

$R_2'$ und $R_3'$ unabhängig voneinander Wasserstoff, Fluor, Brom, $C_1$-$C_4$-Alkyl, $C_1$-$C_4$-Alkoxy, $-CHF_2$ oder Nitro oder

$R_1'$ Propyl oder Isopropyl,

$R_2'$ Chlor und

$R_3'$ Wasserstoff, Halogen, $C_1$-$C_4$-Alkyl, $C_1$-$C_4$-Alkoxy, $C_1$-$C_4$-Halogenalkyl oder Nitro oder

$R_1'$ Cyano und

$R_2'$ und $R_3'$ Wasserstoff oder

$R_1'$ - $COR_4'$ oder $-CH_2-C{\equiv}CH$,

$R_2'$ und $R_3'$ unabhängig voneinander Wasserstoff, Halogen, $C_1$-$C_4$-Alkyl, $C_1$-$C_4$-Alkoxy, $C_1$-$C_4$-Halogenalkyl oder Nitro,

$R_4'$ 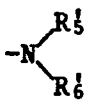 , $C_1$-$C_4$-Alkyl, $C_1$-$C_4$-Halogenalkyl, $C_3$-$C_6$-Cycloalkyl,

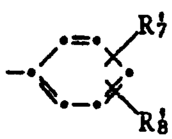 , $-CH_2-$ 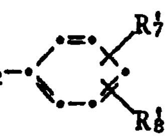 oder Carboxyloxy-$C_1$-$C_4$-alkyl,

$R_5'$ und $R_6'$ unabhängig voneinander Wasserstoff, $C_1$-$C_4$-Alkyl oder $C_1$-$C_4$-Alkoxy und

$R_7'$ und $R_8'$ unabhängig voneinander Wasserstoff, Halogen, $C_1$-$C_4$-Alkyl, $C_1$-$C_4$-Alkoxy oder Nitro oder

$R_1'$ $-CH_2-CH{=}CH_2$ und

$R_2'$ und $R_3'$ unabhängig voneinander Wasserstoff, Halogen, $C_1$-$C_4$-Alkyl, $C_1$-$C_4$-Alkoxy oder Nitro bedeuten.

8. Die Verbindung gemäss Anspruch 7 der Formel

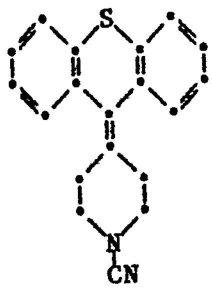

.

9. Die Verbindung gemäss Anspruch 7 der Formel

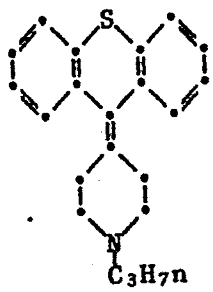

.

10. Die Verbindung gemäss Anspruch 7 der Formel

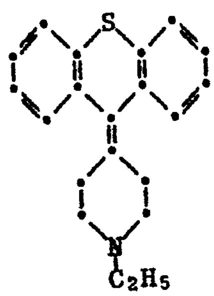

.

11. Die Verbindung gemäss Anspruch 7 der Formel

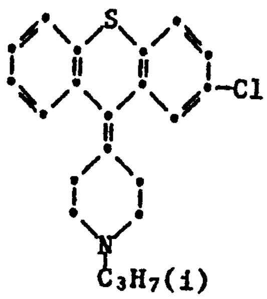

.

12. Die Verbindung gemäss Anspruch 7 der Formel

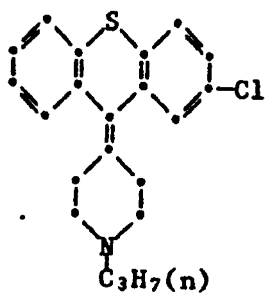

.

13. Die Verbindung gemäss Anspruch 7 der Formel

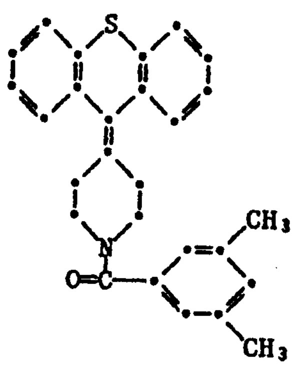

.

0179020

14. Die Verbindung gemäss Anspruch 7 der Formel

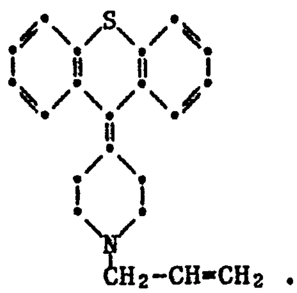

15. Die Verbindung gemäss Anspruch 7 der Formel

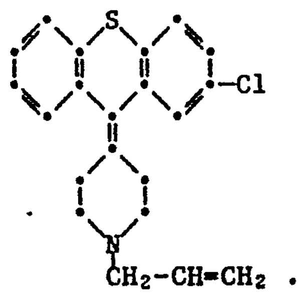

16. Die Verbindung gemäss Anspruch 7 der Formel

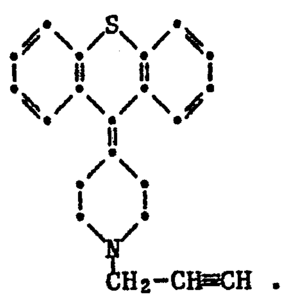

17. Die Verbindung gemäss Anspruch 7 der Formel

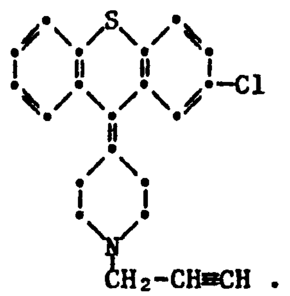

18. Die Verbindung gemäss Anspruch 7 der Formel

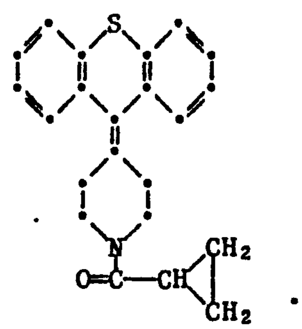

19. Die Verbindung gemäss Anspruch 7 der Formel

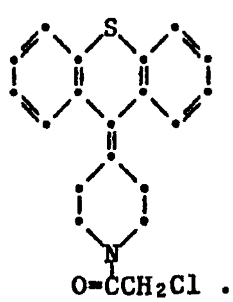

20. Die Verbindung gemäss Anspruch 7 der Formel

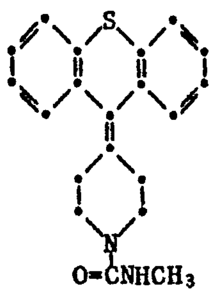

21. Die Verbindung gemäss Anspruch 7 der Formel

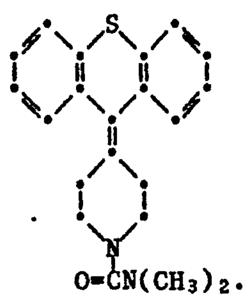

22. Die Verbindung gemäss Anspruch 7 der Formel

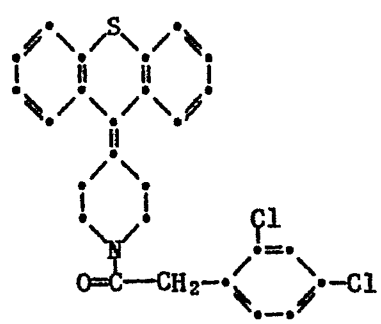

23. Die Verbindung gemäss Anspruch 7 der Formel

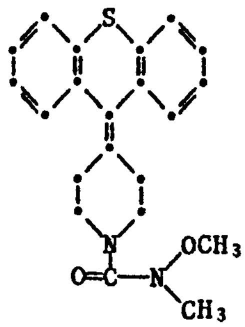

.

24. Die Verbindung gemäss Anspruch 7 der Formel

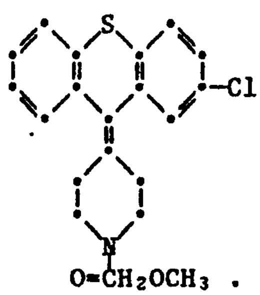

.

25. Die Verbindung gemäss Anspruch 7 der Formel

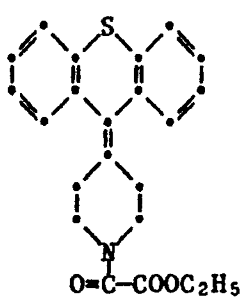

.

26. Die Verbindung gemäss Anspruch 7 der Formel

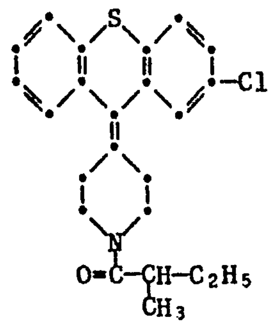

.

27. Die Verbindung gemäss Anspruch 7 der Formel

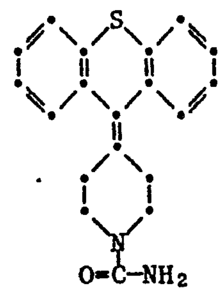

.

28. Die Verbindung gemäss Anspruch 7 der Formel

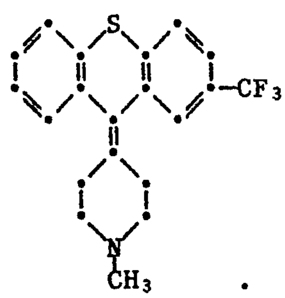

.

29. Die Verbindung gemäss Anspruch 7 der Formel

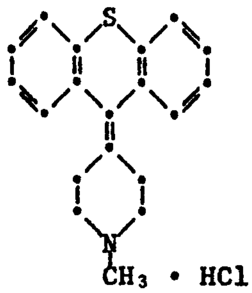

• HCl .

30. Die Verbindung gemäss Anspruch 7 der Formel

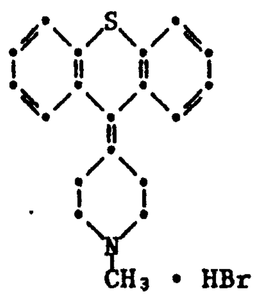

• HBr .

31. Die Verbindung gemäss Anspruch 7 der Formel

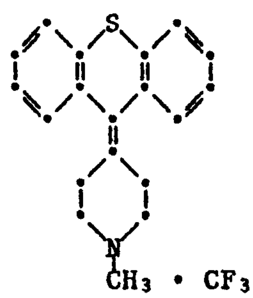

• $CF_3COOH$ .

32. Ein Verfahren zur Herstellung eines Thioxanthen-9-yliden-piperidines der Formeln I und Ia, dadurch gekennzeichnet, dass man die Verbindung der Formel

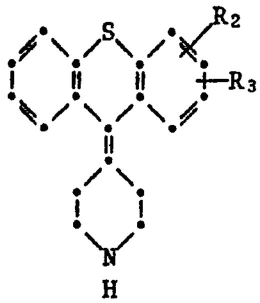 (I) oder 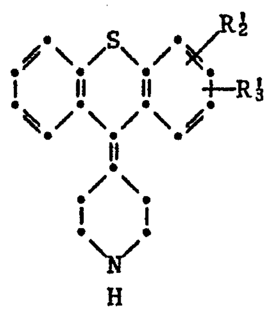

in Gegenwart eines säurebindenden Mittels mit einer Verbindung der Formel $XR_1$ oder $XR_1'$ umsetzt, worin $R_1$, $R_2$ und $R_3$ die im Anspruch 1 und $R_1'$, $R_2'$ und $R_3'$ die im Anspruch 7 angegebene Bedeutung haben und X für ein Halogenatom steht.

33. Die Verwendung von Verbindungen gemäss Anspruch 7 zur Bekämpfung von verschiedenartigen Schädlingen an Tieren und Pflanzen sowie im Boden.

34. Die Verwendung gemäss Anspruch 33 zur Bekämpfung von Insekten und Vertretern der Ordnung Akarina sowie von phytopathogenen Pilzen.

35. Verfahren zur Bekämpfung von Schädlingen an Tieren und Pflanzen, dadurch gekennzeichnet, dass man eine Verbindung gemäss Anspruch 7 auf die Tiere oder die Pflanzen appliziert.

FO 7.5/WH/sm*